# EUROPEAN PATENT SPECIFICATION

(11) **EP 0 868 537 B1**
(45) Date of publication and mention of the grant of the patent: **08.03.2000**
(21) Application number: 96944673.1
(22) Date of filing: 30.12.1996
(51) Int. Cl.: C21C 5/46, G01N 33/20

(54) **METHOD FOR DETERMINATION AND CONTROL OF THE AMOUNT OF NITROGEN DISSOLVED IN METALIC LIQUID PHASES AND DEVICE FOR ITS REALIZATION**
VERFAHREN UND VORRICHTUNG ZUR BESTIMMUNG UND KONTROLLE DES IN FLÜSSIGEN METALLPHASEN GELÖSTEN STICKSTOFFES
PROCEDE DE DETERMINATION ET DE CONTROLE DE LA QUANTITE D'AZOTE DISSOUS DANS DES PHASES LIQUIDES METALLIQUES ET DISPOSITIF POUR SA REALISATION

(30) Priority: 29.12.1995 IT RM950862
(43) Date of publication of application: 07.10.1998
(73) Proprietor: CENTRO SVILUPPO MATERIALI S.p.A., 00129 Roma (IT)
(72) Inventor: BORGIANNI, Carlo, I-00151 Rome (IT); DI DONATO, Antonello, I-65013 Pescara (IT); PISTELLI, Maria, Ilaria, I-00196 Rome (IT)
(74) Representative: Gervasi, Gemma, Dr.
(86) International application number: EP9605860
(87) International publication number: WO9724464

(56) References cited:
- EP-A- 0 024 566
- EP-A- 0 563 447
- EP-A- 0 642 019
- PATENT ABSTRACTS OF JAPAN vol. 011, no. 100 (P-561), 28 March 1987 & JP 61 250556 A (NIPPON KOKAN KK), 7 November 1986,
- PATENT ABSTRACTS OF JAPAN vol. 006, no. 012 (E-091), 23 January 1982 & JP 56 136015 A (SEIKO INSTR & ELECTRONICS LTD), 23 October 1981,
- PATENT ABSTRACTS OF JAPAN vol. 095, no. 006, 31 July 1995 & JP 07 072140 A (NIPPON STEEL CORP;OTHERS: 01), 17 March 1995,

## Description

### FIELD OF INVENTION

The present invention refers to a method for determination and control of the amount of nitrogen dissolved in metallic liquid phases and to the device for its realization.

The invention particularly refers to a device permitting a high measurement rate.

### STATE OF THE ART

It is known that the in-line control of production processes actually has a direct positive influence both on production economy and on the quality of the end product. It is therefore important to have data acquisition systems able to give quick and reliable answers, to permit a real-time monitoring of a process and relevant timely adjustments, if necessary.

In the iron and steel field, it is particularly needed the possibility of a continuous in-line monitoring of the concentration of such elements as nitrogen and hydrogen which are dissolved into the liquid steel from the gaseous environment and negatively influence the mechanical and physical characteristics of the end product.

Such on-line monitoring is described, for instance, in the Japanese patent application No. JP800039365 of March 26, 1980 (publication number JP 56 136 915 of October 26, 1981) in which flue gas leaving a converter is sampled and analyzed for CO, CO₂ and N₂, in order to detect the occurrence of foaming in the converter.

Nitrogen leads to hardening and fragility of ferrous alloys, thus being highly dengerous for steel products such as plates, gas pipes, deep-drawing sheets utilized in car bodies or domestic appliances production, for instance.

The typical phase for nitrogen absorption from the atmosphere is during the steel transfer from the ladle to the mould, through the tundish.

The determination of nitrogen content in liquid steel, directly in the steel shop, and preferably within the mould, could determine in real-time any rise in nitrogen concentration, thus permitting to timely intervene, in well known ways, to lower nitrogen pick-up and eliminate its excess from the metal bath.

In the control of metallic materials production, particularly during the step in which such materials are in the liquid state, up to now the determination of dissolved chemical species, especially gases, was made through quickly solidified metal samples sent to the laboratory for analysis. Such a method, though accurate with errors within 5%, is not a satisfying one, due to excessive delay for the answer, tipically of 10-30 minutes, which does not allows for timely bath composition adjustments.

Devices for sequentially and/or continuously determining concentrations of carbon, hydrogen and nitrogen in molten steel are known from i.a. EP-A-642 019 and EP-A-563 447.

A device for nitrogen determination in the liquid steel is commercialized under the NITRIS trademark by Heraeus-ElectroNite International N.V. The measurement method requires the immersion into the liquid bath of a probe delivering an inert carrier gas, specifically helium. The nitrogen dissolved into the liquid steel is then divided, according to known laws, between metal and gas, and in the latter tends to a concentration, according to the Henry-Sieverts law, in equilibrium with, and proportional to, the one within the liquid bath.

From the gas coming out from the bath, and containing all the chemical species extracted from the same, all the species interfering with the nitrogen analysis are eliminated through specific molecular sieves, and the purified gas is sent to the analysis made utilizing known apparatuses for thermal conductivity determination.

The probe for introducing into the liquid bath a specific amount of helium, and for sampling it and send it to the analysis, is a disposable one and can give an answer in about 90 seconds, with a declared accuracy of 10%.

It is possible to enhance this accuracy utilizing a well known artifice. After a first measure, a new extraction operation is started utilizing as carrier gas a mixture of helium and nitrogen in which the nitrogen content corresponds to the one previously determined, and the measure is repeated. If the new measure is identical to the previous one, the latter was correct; if a nitrogen content is determined higher than the previous one, the actual nitrogen content of the bath is higher than the one obtained in the previous measure, while if the newly determined content is lower, the bath has a lower nitrogen content. The above artifice permits to obtain an accuracy of about 5%. Such probes for the nitrogen determination, though representing an interesting progress with respect to the classic technique of laboratory analysis, still maintain even important drawbacks:
- are of the disposable kind, allowing only a single determination at a time, thus not permitting a continuous monitoring;
- it is practically impossible to introduce the probes into the mould with the necessary high cadence, first of all because it is forbidden to stay under the ladle during the casting and then because, even if it should be possible, the presence of casting powders and of semiliquid slag on the bath surface into the mould would make it highly difficult to correctly introduce the probe at the location and within the desired time;
- the measure time of 90 seconds is still too long for some kind of intervention, and anyway it have to be strictly maintained: in fact, different residence times of the probe into the bath would make meaningless the measure;
- in the nitrogen determination, it is necessary to utilize as carrier gas helium, which is very costly and usually not available in a steel shop.

It is still unsolved the problem of the quick and accurate determination of the nitrogen content in liquid metal baths, in a continuous way or at least with a very short time interval from measure to measure.

### SUMMARY OF THE INVENTION

The above problems are solved by the present invention, comprising in a first aspect thereof a device permitting, when placed in a steel producing plant and for instance in a tundish or in a continuous casting mould, the determination of nitrogen content directly from the liquid phase. Such a device can point out possible nitrogen pick-up in real-time, thus permitting to immediately intervene.

Another aspect of present invention is a method for the determination and control of the nitrogen content of metal baths by means of such a device.

The method, resp. the device according to the invention for determination and control of dissolved nitrogen amounts in molten metal baths, is defined by the technical features of claim 1, resp. of independent claim 7. Preferred embodiments of the invention are defined in the dependent claims.

Other objects of present invention shall become evident from the following detailed description of the invention.

### BRIEF DESCRIPTION OF THE FIGURES

Fig. 1 is a general scheme of a device according to the present invention;
Fig. 2 schematically shows the reactor according to Fig. 1 for nitrogen oxidation;
Fig. 3 is an enlarged schematic longitudinal section of a sensor particularly suited to measure the NO₂ content of the extracted gas.

### DETAILED DESCRIPTION OF THE INVENTION

For a better understanding, the present invention will be described starting from its realization method, which will simplify the following description and understanding of the relevant device.

To each phase of the process a part of the device is logically and operatively connected, as apparent from the following description.

The method for the determination and control of nitrogen content in metal baths, according to present invention, comprises the following steps:
- introducing into the liquid metal (in the following description also called metal bath or simply bath) a known quantity of inert carrier gas, for instance argon also mixed with other gases such as nitrogen, to promote a nitrogen exchange between the bath and the carrier gas;
- withdrawing the carrier gas passed through the bath and containing nitrogen, adding to it a known quantity of oxygen and homogenizing the gas mixture thus obtained;
- oxidizing the nitrogen contained in said gaseous mixture to nitrogen oxides, mainly NO₂ and small quantities of NO;
- measuring the NO₂ content in said mixture and correlating it to the nitrogen content in the bath;
- charging said analysys data in a computer and utilizing them control the nitrogen content in the metal bath.

The carrier gas flow rate into the metal bath is comprised between 3 and 30 litres per hour.

The oxygen flow rate to be added to the carrier gas passed through the bath is from 40 to 70% of flow rate of carrier gas and the mixture is throughly homogenized and then treated to catalyze the nitrogen combustion to NO₂, and small quantities of NO.

Preferably, the homogenized gaseous mixture is subjected to electric sparks of between 3000 to 8000 V, to oxidize nitrogen to mainly NO₂.

The NO₂ content of the thus obtained gaseous mixture is measured with known methods, and is correlated to the nitrogen content in the liquid metal bath, and the thus obtained concentration is utilized according to known methods to adjust, if necessary, the nitrogen content in the metal bath and hence to check and control the final quality of the products obtained from said metal bath.

The production of small quantities of NO during the nitrogen combustion does not invalidate the method. In fact, the NO quantity is very small and its measure can be considered within the background noise, thus not significantly influencing the NO₂ measure. To avoid "interpollution" of different measurements, the NO₂ containing gas is sent to the analyzer in a non-continuous way, at regular time intervals as known volumes, utilizing an inert carrier gas, e.g. argon. Between a gas volume to be analyzed and the next one, the analyzer is fed with pure carrier gas, to purge any residual quantity of NO₂. The gas volume to be analyzed, sent to the analyzer, is comprised between 0,3 amd 5 ml, while the carrier gas flow-rate during this step is between 5 and 20 l/h.

With the utilized experimental apparatus, it is possible to perform quick measurements, typically a measure in 15-25 s, with an error lesser than 10%, improvable to less than 5% utilizing a double-measure system, as hereinafter specified.

The measures cadence (2-4 measures per minute, instead of 0,6 measures per minute of known methods), though not permitting continuous measures, is however sufficiently quick to permit timely actions to control the nitrogen content in the liquid bath.

The device according to present invention for the embodiment of the above method, comprises the combination in cooperation relationship of:
- means for bubbling and picking-up a gas, enabling to inject an inert gas into a metal bath, thus realizing a nitrogen exchange between said bath and the injected gas, and to collect the obtained gas mixture;
- means for extracting said gas mixture from said bubbling and picking-up means;
- means for adding desired quantities of oxygen to said extracted mixture and means for homogenizing the gaseous mixture thus obtained;
- means to subject the nitrogen contained in said homogenized gaseous mixture to a catalyzed oxidation;
- means for measuring the NO₂ content in said oxidated gaseous mixture;
- means to acquire data relating to NO₂ content in the oxidized gaseous mixture, transforming them in nitrogen content in the metal bath and utilizing them to monitor and modify, if necessary, the nitrogen content of the bath.

The means for bubbling and picking-up a gas substantially comprise an element, for instance of an elongated tubular form, for picking-up the introduced gas, having a first conduit with a downwardly facing opening for the admission into said element of the gas to be bubbled through the bath, and a second conduit connected to said means for extracting the collected gas, for instance consisting in a rotary pump.

The means for adding to the extracted gas known quantities of oxygen and for homogenizing the thus obtained mixture consist in an oxygen tank having means for controlling, measuring and purifying the outcoming oxygen flow, and in a conduit for admitting gas into a chamber, having openings for amitting and extracting gas, in which the extracted gas and oxygen are admitted. Said chamber is provided with a plurality of projections, for instance suitably located walls, to enhance the turbulence of the gases passing through said chamber and thus obtain a homogeneous gas mixture.

Said means to oxidize the nitrogen in said homogenized gaseous mixture consist, for instance, in a reactor internally provided with means to subject the gaseous mixture containing nitrogen and oxygen to a catalytic oxidation reaction of the nitrogen, mainly to NO₂. In said reaction, the oxidation efficiency of tne nitrogen (i.e. the ratio NO₂/NO) is constant.

Preferably, said means to subject the gas to a catalytic oxidation consist in at least a couple of electrodes, e.g. platinum ones, between which a tension is established sufficiently high to establish an electric arc.

Said means for measuring the NO₂ content in the gases coming out the reactor can be any sensor; preferably, said sensor comprises (i) a first conduit in which the gaseous mixture containing a component to be measured flows with laminar motion, (ii) a second conduit in which a carrier liquid, in which said component is soluble, flows with laminar motion, (iii) a membrane permeable only to said component and constituting a separating wall between said first and said second conduits, and (iv) a reference electrode and a measure electrode, sensing the concentration of said component, both placed in said second conduit.

The linear speed of the carrier liquid on the semipermeable membrane is comprised between 10⁻¹ and 10⁻⁵ m/s while the gas linear speed on the membrane is lesser than 5 m/s.

The reference electrode is placed in said second conduit before said membrane, thus being always immersed in clean carrier liquid and therefore maintaining constant its potential, which is the reference potential. Said reference electrode has an wetted surface greater than the one of the measure electrode, preferably from 2 to 5 times greater.

The semipermeable membrane is made with a porous hydrofobic polymer, with a mead pore diametre of 1 micrometre.

The gaseous mixture flows within the space between first and second conduits, and the carrier liquid flows within said second conduit in countercurrent with respect to the gaseous mixture, as a thin layer over the semipermeable membrane; its flow rate is comprised between 0,005 and 0,1 ml/s, preferably between 0,01 and 0,03 ml/s, its thickness being comprised between 2 and 0,05 mm, preferably between 1 and 0,1 mm.

Said means to acquire data concerning the NO₂ content in the gas and for correlating them to the nitrogen content in the metal bath comprise a computer provided with a standard program and with specific calibration curves, which can be obtained in any known way.

It is to be noted that though the above device, particularly the sensor, is described only with reference to the analysis of NO₂, it can be easily utilized for the analysis of other species, of both acid and basic nature.

Coming now to the enclosed Figures, the means for bubbling and picking-up the gas comprise a refractory collecting vessel (20) resistant to thermal shocks, preferably in silicon carbide, provided with a conduit (21), having flow controlling means (23), for introducing a carrier gas and provided also with a second conduit (22) to withdraw said gas from said vessel. The carrier gas, typically argon, flows within conduit (22) coming from a tank (11), and its flow rate is measured and controlled by a regulating flowmetre (13).

In operation, the vessel (20) is immersed into the metal bath with its opening facing downwardly, to a level comprised from 100 to 300 mm, and valve (23) is opened, allowing the carrier gas to bubble within the bath, activating the nitrogen exchange between bath and gas.

The gas coming out of the bath and enriched with nitrogen is then sent, by means of a pump (not shown), and of conduit (22) to the mixer/homogenizer (30) into which is mixed with oxygen coming from tank (31) through conduit (32) with a desired flow rate, measured and controlled by flowmetre (33). The mixer/homogenizer (30) comprises a sealed chamber internally provided with deflecting walls, helical paths and the like permitting to homogenize the gaseous mixture consisting of argon, nitrogen and oxygen which is then sent to the reactor (40). The latter (Giggs 1 and 2) comprises a tubular chamber, containing at least a pair of electrodes (42) facing to each other; between said electrodes and port (45) for the immission of the homogenized gaseous mixture into chamber (40) a narrowing (44) is placed, preferably having a conical form, to send the gaseous mixture flow exactly in the space existing between the electrodes (42), in which an electric sparks are generated through a generator (41), as described with reference to the embodiment method. Thus, the energy of the electric sparks catalyzes the nitrogen combustion, mainly to NO₂. The generation of NO during the above process does not impair the validity of the measures, in that NO concentration is very small and the NO₂/NO ratio is practically constant.

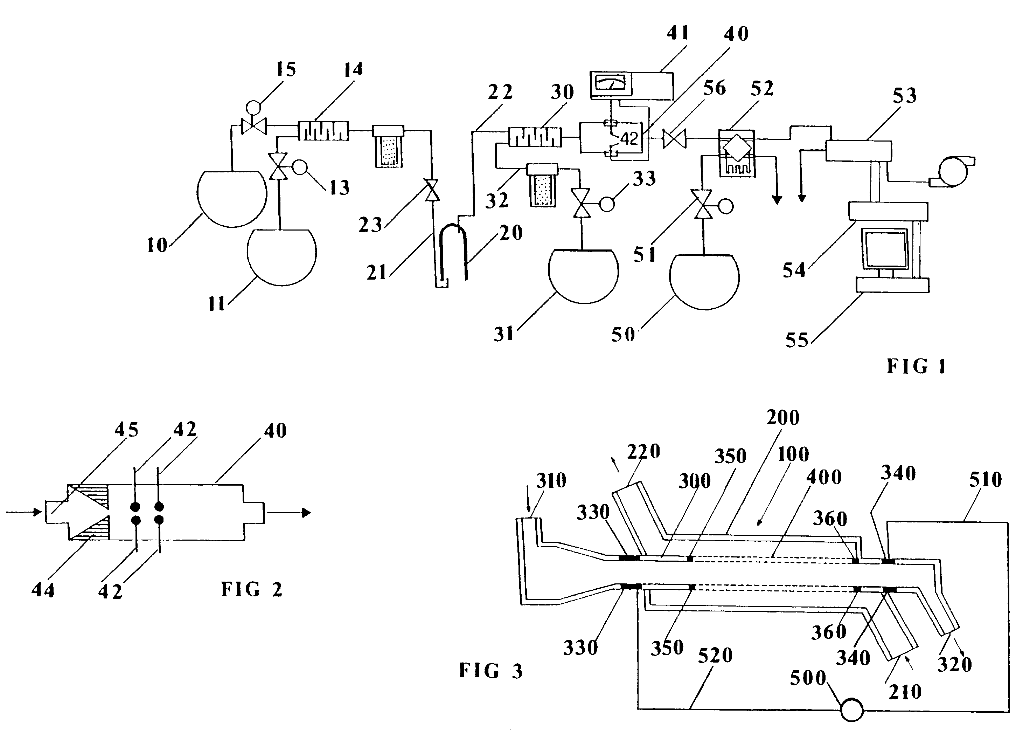

During the measures the NO₂ containing mixture, thus obtained, is sent to the analysis device (53), which can be of the kind illustrated in Fig. 3. This device (100) comprises: (i) a first conduit (200) having an inlet opening (210) and a discharge opening (220), (ii) a second conduit (300) also having an inlet opening (310) and a discharge opening (320), and comprising a membrane (400) semipermeable to gases the extremities (350, 360) of which are connected to metallic tubular elements containing (iii) a pair of reference (330) and measure (340) electrodes.

Conduit (300) is internal and coaxial to conduit (200), and its metallic extremities, preferably made in stainless steel, comprise a first oxidized and stabilized zone (330), acting as reference electrode, and a second oxidized and stabilized zone (340), acting as measure electrode. Cables (510, 520) respectively connect electrodes (330) and (340) to a measure instrument (500), in this case a millivoltmeter.

The oxidized gaseous mixture is periodically sent to the analyzer as constant and known volumes controlled by a flowmetre (56) and by a four-ways cock (52), utilizing a carrier gas, argon for instance, erogates at a constant rate by a reservoir (50) through a flowmetre (51).

An important advantage of the device according to present invention is that vessel (20) can be permanently placed into the liquid bath, e.g. into the continuous casting mould, where can work during the entire casting process. This means that vessel (20) can be put in place before starting the casting operations (then easily and in complete safety for the operators) and continuously works for many hours without modifying the operating conditions of the plant.

The device according to the invention can pinpoint on the line and within 15-25 seconds any unduly high nitrogen content into the bath, allowing to timely operate to reduce said nitrogen content thus avoiding a downgrading of the end product.

The device according to the invention can measure NO₂ contents even smaller than 1 ppm, on very small gas volumes.

According to the present invention, the measure error of the nitrogen content in the metal bath is around 10%, depending on the uncertainties introduced by bath temperature variations and bath content of such elements as sulphur and oxygen, which can influence the exchange cinetics of nitrogen between metal bath and gas.

It is possible to improve such figures utilizing a double-analysis technique, according to which after a first measure, obtained as above described, a second one is made, passing through the bath a nitrogen-argon mixture in which the nitrogen, coming from tank (10) and measured by flowmeter (15), has a content corresponding to the one in equilibrium with the nitrogen in the bath, known from the first measure; the mixture is homogenized in the mixer (14).

If in said second measure the nitrogen content in the mixture passed through the bath is really in equilibrium with the bath, there will be no change in the nitrogen content in the gaseous mixture, and the measure will remain unchanged. On the other hand, should the measured concentrations be different, it is possible to desume, in a known way, the nitrogen concentration in equilibrium with the bath through solution of systems of differential cynetics equations. From this value, the Henry-Sieverts law allows to obtain the real concentration of nitrogen in the bath.

It is thus possible to enhance the accuracy of the measures, at the expense of a doubling of the time necessary for each measure; however, a cadence of less than 50 seconds can be considered acceptable in most cases.

## Claims

1. Method for the measure and the control of the nitrogen content in liquid metal baths, comprising in cooperative relationship the following steps:
- introducing into the liquid metal a metered quantity of inert carrier gas, for instance argon optionally mixed with other gases such as nitrogen, and letting it bubble through the bath to promote a nitrogen exchange between the bath and the carrier gas, and then collecting the thus exchanged nitrogen/carrier gas mixture ;
- withdrawing the collected gas, adding to it a known quantity of oxygen and homogenizing the gas mixture thus obtained;
- oxidizing the nitrogen contained in said gaseous mixture to nitrogen oxides, mainly NO₂ including small quantities of NO;
- measuring the NO₂ content in said mixture and correlating it to the nitrogen content in the bath;
- charging said analysis data in a computer and utilizing them for control of the nitrogen content in the metal bath.

2. Method according to clain 1, characterized in that the carrier gas flow rate into the metal bath is comprised between 3 and 30 litres per hour.

3. Method according to any one of the above claims, characterized in that the oxygen flow rate to be added to the carrier gas passed through the bath is from 40 to 70% of flow rate of carrier gas and in that the mixed gases are thoroughly homogenized in a mixing chamber.

4. Method according to any one of the preceding claims, characterized in that the homogenized gaseous mixture comprising nitrogen and oxygen is treated to catalyze the nitrogen combustion mainly to NO₂.

5. Method according to claim 4, characterized in that the homogenized gaseous mixture is subjected to electric sparks of between 3000 to 8000 V, to catalyze the oxidation of nitrogen to mainly NO₂.

6. Method according to any one of the preceding claims, characterized in that the gaseous mixture containing NO₂ is sent to an analyzer for the determination, according to known ways, of the NO₂ content which, in turn, is correlated to the nitrogen content in the liquid metal bath, and in that the thus obtained concentration is utilized according to known methods to adjust the nitrogen content in the liquid metal.

7. Device for performing the method according to claim 1, comprising the combination in cooperative relationship of:
- means (20, 21, 23) including a probe (20) for bubbling and picking-up a gas, enabling to inject a metered quantity of an inert carrier gas into a metal bath, thereby realizing a nitrogen exchange between said bath and the injected gas, and to collect a thus exchanged nitrogen carrier gas mixture ;
- means (22) for extracting said gas mixture from said bubbling and picking-up means;
- means (31, 32, 33) for adding predetermined quantities of oxygen to said extracted mixture and means (30) for homogenizing the gaseous mixture thus obtained;
- means (40, 41, 42) to oxidize mainly to NO₂ the nitrogen contained in said homogenized gaseous mixture;
- means (53, 100) for measuring the NO₂ content in said oxidized gaseous mixture;
- means (54, 55) for processing the above acquire data and relating them to the nitrogen content in the bath.

8. Device according to claim 7, characterized in that the means for bubbling and picking-up a gas comprise an elongated refractory vessel element (20) provided with an internal space and having a first opening at one of its extremities, a first conduit (21) for the admission into said space of an inert carrier gas and having a second opening provided with second conduit means (22) for extracting an exchanged gas means to control the gas flow in said conduit means (21, 22).

9. Device according to claims 7 and 8, characterized in that said means for adding to the extracted gas predetermined quantities of oxygen and for homogenizing the thus obtained mixture consist in an oxygen tank (31) having means (33) for controlling and measuring the outcoming oxygen flow, and in a mixing chamber (30), having inlet openings for admitting the extracted gas and oxygen, said chamber being provided with a plurality of suitably located projections to enhance the turbulence of the admitted gases passing through said chamber, thereby obtaining a homogeneous gas mixture.

10. Device according to any of claims from 7 to 9, characterized in that said means to oxidize the nitrogen in said homogenized gaseous mixture comprise a tubular reactor (40) internally provided with means (42) to subject the gaseous mixture containing nitrogen and oxygen to a catalytic oxidation reaction of the nitrogen into mainly NO₂.

11. Device according to claim 10, characterized in that said means (42) to subject the gas to a catalytic oxidation consist in at least a couple of electrodes between which a tension is established sufficiently high to establish an electric arc.

12. Device according to any one of claims from 7 to 11, characterized in that said means (53, 100) for measuring the NO₂ content in the gases coming out the oxidation reactor comprise (i) a first conduit (200) in which the gaseous mixture containing a component to be measured flows with laminar motion, and provided with an inlet opening (210) and a discharge opening (220); (ii) a second conduit (300), adjacent to the first one, through which a carrier liquid, in which said component is soluble, flows with laminar motion in countercurrent with said gaseous mixture, said second conduit having an inlet opening (310) and a discharge opening (320); (iii) a membrane (400) in a hydrophobic polymer permeable only to said component and constituting a separating wall between said first and said second conduits, and (iv) a couple of reference (330) and measure (340) electrodes, respectively, placed in said conduit (300) at the extremities of membrane (400).

13. Device according to any one of claims from 7 to 12, characterized in that said second conduit (300) comprises stainless steel zones, carrying a reference electrode (33) consisting of a first oxidized and stabilized surface part, and a measure electrode (340) consisting of a second oxidized and stabilized surface part, said measure electrode (340) being from 20% to 50% longer than the reference one (330), from 2 to 5 times greater.

## Patentansprüche

1. Verfahren zur Messung und Kontrolle des Stickstoffgehaltes in flüssigen Metallbädern, das die folgenden miteinander kooperierenden Stufen umfaßt:
- Einführen einer abgemessenen Menge eines inerten Trägergases wie Argon, gegebenenfalls im Gemisch mit anderen Gasen wie Stickstoff, in das flüssige Metall und Durchperlenlassen desselben durch das Metallbad, um einen Stickstoffaustausch zwischen dem Metallbad und dem Trägergas zu fördern, und anschließendes Sammeln der auf diese Weise ausgetauschten Stickstoff/Trägergas-Mischung;
- Abziehen des gesammelten Gases, Zugabe einer bekannten Menge Sauerstoff und Homogenisieren der so erhaltenen Gasmischung;
- Oxidieren des in der genannten Gasmischung enthaltenen Stickstoffs zu Stickstoffoxiden, hauptsächlich NO₂ einschließlich geringer Mengen NO;
- Bestimmen des NO₂-Gehaltes in der genannten Mischung und Aufstellen einer Korrelation zwischen diesem und dem Stickstoff-Gehalt in dem Bad; und
- Eingeben der genannten Analysendaten in einen Computer und Verwendung derselben zur Kontrolle des Stickstoff-Gehaltes in dem Metallbad.

2. Verfahren nach Anspruch 1, dadurch gekennzeichnet, daß die Strömungsgeschwindigkeit des in das Metallbad eingeführten Trägergases zwischen 3 und 30 l/h liegt.

3. Verfahren nach einem der vorhergehenden Ansprüche, dadurch gekennzeichnet, daß die Strömungsgeschwindigkeit des dem Trägergas zuzuführenden Sauerstoffs, das durch das Metallbad hindurchgeführt wird, 40 bis 70 % der Strömungsgeschwindigkeit des Trägergases beträgt und daß die gemischten Gase in einer Mischkammer gründlich homogenisiert werden.

4. Verfahren nach einem der vorhergehenden Ansprüche, dadurch gekennzeichnet, daß die homogenisierte Gasmischung, die Stickstoff und Sauerstoff enthält, behandelt wird, um die Stickstoffverbrennung, hauptsächlich zu NO₂, zu katalysieren.

5. Verfahren nach Anspruch 4, dadurch gekennzeichnet, daß die homogenisierte Gasmischung einer elektrischen Lichtbogenentladung zwischen 3000 und 8000 V unterworfen wird, um die Oxidation von Stickstoff, hauptsächlich zu NO₂, zu katalysieren.

6. Verfahren nach einem der vorhergehenden Ansprüche, dadurch gekennzeichnet, daß die Gasmischung, die NO₂ enthält, in einen Analysator eingeführt wird zur Bestimmung des NO₂-Gehaltes auf an sich bekannte Weise, der seinerseits zu dem Stickstoff-Gehalt in dem flüssigen Metallbad in Korrelation gesetzt wird, und daß die auf diese Weise erhaltene Konzentration dazu verwendet wird, den Stickstoff-Gehalt in dem flüssigen Metall nach bekannten Methoden einzustellen.

7. Vorrichtung zur Durchführung des Verfahrens nach Anspruch 1, die eine Kombination der folgenden miteinander kooperierenden Einrichtungen aufweist:
- eine Einrichtung (20, 21, 23) einschließlich einer Sonde (20) zum Hindurchperlenlassen und Aufnehmen eines Gases, die ermöglicht das Einleiten einer abgemessenen Menge eines inerten Trägergases in ein Metallbad, um dadurch einen Stickstoffaustausch zwischen dem genannten Metallbad und dem eingeleiteten Gas zu bewirken, und das Sammeln der auf diese Weise ausgetauschten Stickstoff/Trägergas-Mischung;
- eine Einrichtung (22) zum Extrahieren der genannten Gasmischung aus der genannten Gaseinleitungs- und Gasaufnahme-Einrichtung;
- eine Einrichtung (31, 32, 33) zur Zugabe von vorgegebenen Mengen an Sauerstoff zu der genannten extrahierten Mischung und eine Einrichtung (30) zum Homogenisieren der so erhaltenen Gasmischung;
- eine Einrichtung (40, 41, 42) zum Oxidieren des in der genannten homogenisierten Gasmischung enthaltenen Stickstoffs, hauptsächlich zu NO₂;
- eine Einrichtung (53,100) zur Bestimmung des NO₂-Gehaltes in der genannten oxidierten Gasmischung; und
- eine Einrichtung (54, 55) zur Verarbeitung der wie oben gewonnenen Daten und zur Aufstellung einer Korrelation zwischen diesen und dem Stickstoff-Gehalt in dem Bad.

8. Vorrichtung nach Anspruch 7, dadurch gekennzeichnet, daß die Einrichtung zum Einperlenlassen und Aufnehmen eines Gases umfaßt ein längliches feuerfestes Behälter-Element (20), das ausgestattet ist mit einem inneren Hohlraum und das an einem seiner Enden eine erste Öffnung aufweist, die mit einer ersten Rohrleitung (21) zur Einführung eines inerten Trägergases in den genannten Hohlraum in Verbindung steht, und das eine zweite Öffnung aufweist, die mit einer zweiten Rohrleitung (22) in Verbindung steht, zum Extrahieren eines ausgetauschten Gases, und eine Einrichtung zur Kontrolle des in die genannten Rohrleitungen (21, 22) einströmenden Gases.

9. Vorrichtung nach Anspruch 7 und 8, dadurch gekennzeichnet, daß die genannte Einrichtung zur Zugabe von vorgegebenen Mengen an Sauerstoff zu dem extrahierten Gas und zur Homogenisierung der auf diese Weise erhaltenen Mischung besteht aus einem Sauerstofftank (31), der eine Einrichtung (33) zur Kontrolle und Messung des ausströmenden Sauerstoffstroms aufweist, und einer Mischkammer (30), die Einlaßöffnungen zum Einführen des extrahierten Gases und des Sauerstoffs aufweist, wobei die genannte Kammer mit einer Vielzahl von in geeigneter Weise angeordneten Vorsprüngen ausgestattet ist, um die Turbulenz der eingeführten Gase beim Passieren der genannten Kammer zu verstärken, so daß man eine homogene Gasmischung erhält.

10. Vorrichtung nach einem der Ansprüche 7 bis 9, dadurch gekennzeichnet, daß die genannte Einrichtung zum Oxidieren des Stickstoffs in der genannten homogenisierten Gasmischung einen rohrförmigen Reaktor (40) umfaßt, der innen mit Einrichtungen (42) ausgestattet ist, um die Stickstoff und Sauerstoff enthaltende Gasmischung einer katalytischen Oxidationsreaktion des Stickstoffs zu hauptsächlich NO₂ zu unterwerfen.

11. Vorrichtung nach Anspruch 10, dadurch gekennzeichnet, daß die genannte Einrichtung (42), in der das Gas einer katalytischen Oxidation unterzogen wird, besteht aus mindestens einem Paar Elektroden, an die eine ausreichend hohe Spannung angelegt wird, um einen elektrischen Lichtbogen zu erzeugen.

12. Vorrichtung nach einem der Ansprüche 7 bis 11, dadurch gekennzeichnet, daß die genannte Einrichtung (53, 100) zur Bestimmung des NO₂-Gehaltes in den Gasen, die aus dem Oxidationsreaktor herausströmen, umfaßt (i) eine erste Rohrleitung (200), in der die Gasmischung, die eine zu messende Komponente enthält, in laminarer Strömung fließt und die mit einer Einlaßöffnung (210) und einer Austragsöffnung (220) ausgestattet ist; (ii) eine zweite Rohrleitung (300), die benachbart zu der ersten Rohrleitung angeordnet ist, durch die eine Trägerflüssigkeit, in der die genannte Komponente löslich ist, in laminarer Strömung fließt im Gegenstrom zu der genannten Gasmischung, wobei die genannte zweite Rohrleitung eine Einlaßöffnung (310) und eine Austragsöffnung (320) aufweist; (iii) eine Membran (400) aus einem hydrophoben Polymer, das nur für die genannte Komponente durchlässig ist und eine Trennwand zwischen der genannten ersten Rohrleitung und der genannten zweiten Rohrleitung darstellt, und (iv) ein Paar Bezugselektroden (330) bzw. Meßelektroden (340), die in der genannten Rohrleitung (300) an den Enden der Membran (400) angeordnet sind.

13. Vorrichtung nach einem der Ansprüche 7 bis 12, dadurch gekennzeichnet, daß die genannte zweite Rohrleitung (300) Zonen aus rostfreiem Stahl umfaßt, die eine Bezugselektrode (33), bestehend aus einem ersten oxidierten und stabilisierten Oberflächenteil, und eine Meßelektrode (340), bestehend aus einem zweiten oxidierten und stabilisierten Oberflächenteil, tragen, wobei die genannte Meßelektrode (340), die um 20 bis 50 % länger ist als die Bezugselektrode (330), 2 bis 5 mal größer ist.

## Revendications

1. Procédé pour mesurer et contrôler la teneur en azote dissous dans des bains de métaux liquides, comprenant, selon une relation de coopération, les étapes suivantes consistant à :
- introduire dans le métal liquide une quantité dosée d'un gaz porteur inerte, par exemple de l'argon, mélangé facultativement à d'autres gaz tels que de l'azote, et laisser barboter le gaz dans le bain pour favoriser un échange d'azote entre le bain et le gaz porteur, puis collecter le mélange azote échangé/gaz porteur;
- retirer le gaz collecté, lui ajouter une quantité connue d'oxygène et homogénéiser le mélange gazeux ainsi obtenu;
- oxyder l'azote contenu dans ledit mélange gazeux pour obtenir des oxydes d'azote, principalement du NO₂ incluant de faibles quantités de NO;
- mesurer la teneur en NO₂ dudit mélange et la corréler à la teneur en azote dans le bain;
- charger lesdites données d'analyse dans un ordinateur et les utiliser pour commander la teneur en azote dans le bain de métal.

2. Procédé selon la revendication 1, caractérisé en ce que le débit du gaz porteur introduit dans le bain de métal est compris entre 3 et 30 litres par heure.

3. Procédé selon l'une quelconque des revendications précédentes, caractérisé en ce que le débit d'oxygène devant être ajouté au gaz porteur traversant le bain est compris entre 40 et 70 % du débit du gaz porteur et en ce que les gaz mélangés sont soigneusement homogénéisés dans une chambre de mélange.

4. Procédé selon l'une quelconque des revendications précédentes, caractérisé en ce que le mélange gazeux homogénéisé comprenant de l'azote et de l'oxygène est traité de manière à catalyser la combustion de l'azote pour obtenir principalement du NO₂.

5. Procédé selon la revendication 4, caractérisé en ce qu'on soumet le mélange gazeux homogénéisé à des étincelles électriques comprises entre 3000 et 8000 V pour catalyser l'oxydation de l'azote pour former principalement du NO₂.

6. Procédé selon l'une quelconque des revendications précédentes, caractérisé en ce que le mélange gazeux contenant du NO₂ est envoyé à un analyseur pour la détermination, selon des moyens connus, de la teneur en NO₂ qui a son tour est corrélée à la teneur en azote dans le bain de métal liquide, et en ce que la concentration ainsi obtenue est utilisée selon des procédés connus pour régler la teneur en azote dans le métal liquide.

7. Dispositif pour la mise en oeuvre du procédé selon la revendication 1, comprenant la combinaison selon une liaison de coopération entre :
- des moyens (20,21,23) comprenant une sonde (20) servant à faire barboter et collecter un gaz, en permettant l'injection d'une quantité dosée du gaz porteur inerte dans un bain métallique, en réalisant de ce fait un échange d'azote entre ledit bain et le gaz injecté, et collecter un mélange azote ainsi échangé/gaz porteur;
- les moyens (22) pour extraire ledit mélange gazeux desdits moyens de barbotage et de collecte;
- des moyens (31,32,33) pour ajouter des quantités prédéterminées d'oxygène audit mélange extrait et des moyens (30) pour homogénéiser le mélange gazeux ainsi obtenu;
- des moyens (40,41,42) pour oxyder principalement en NO₂ l'azote contenu dans ledit mélange gazeux homogénéisé;
- des moyens (53,100) pour mesurer la teneur en NO₂ dans ledit mélange gazeux oxydé;
- des moyens (54,55) pour traiter les données acquises indiquées précédemment et les associer à la teneur en azote dans le bain.

8. Dispositif selon la revendication 7, caractérisé en ce que les moyens de barbotage et de collecte d'un gaz comprennent un élément allongé d'enceinte réfractaire (20) pourvu d'un espace interne et possédant une première ouverture à l'une de ses extrémités, un premier conduit (21) pour l'admission, dans ledit espace, d'un gaz porteur inerte, et possédant une seconde ouverture équipée de seconds moyens formant conduit (22) pour extraire un gaz échangé, et des moyens pour régler la circulation du gaz dans lesdits moyens formant conduits (21,22).

9. Dispositif selon les revendications 7 et 8, caractérisé en ce que lesdits moyens pour ajouter des quantités prédéterminées d'oxygène au gaz extrait et pour homogénéiser le mélange ainsi obtenu se composent d'un réservoir d'oxygène (31) comprenant des moyens (33) pour régler et mesurer le débit d'oxygène sortant, et d'une chambre de mélange (30) comportant des ouvertures d'entrée pour admettre le gaz extrait et l'oxygène, ladite chambre étant pourvue d'une pluralité de parties saillantes positionnées de façon appropriée pour améliorer la turbulence des gaz admis traversant ladite chambre, qui permet d'obtenir un mélange gazeux homogène.

10. Dispositif selon l'une quelconque des revendications 7 à 9, caractérisé en ce que lesdits moyens pour oxyder l'azote dans ledit mélange gazeux homogénéisé comprennent un réacteur tubulaire (40) équipé intérieurement de moyens (42) pour soumettre le mélange gazeux contenant de l'azote et de l'oxygène à une réaction d'oxydation catalytique de l'azote principalement en du NO₂.

11. Dispositif selon la revendication 10, caractérisé en ce que lesdits moyens (42) servant à soumettre le gaz à une oxydation catalytique sont constitués par au moins un couple d'électrodes, entre lesquelles une tension est établie à une valeur suffisamment élevée pour former un arc électrique.

12. Dispositif selon l'une quelconque de revendications 8 à 11, caractérisé en ce que lesdits moyens (53,100) servant à mesurer la teneur en NO₂ dans les gaz sortant du réacteur d'oxydation comprennent (i) un premier conduit (200), dans lequel le mélange gazeux contenant un constituant à mesurer circule selon un déplacement laminaire, et qui est pourvu d'une ouverture d'entrée (210) et d'une ouverture d'évacuation (220); (ii) un second conduit (300), qui est adjacent au premier conduit et dans lequel un liquide porteur, dans lequel ledit constituant est soluble, circule avec un déplacement laminaire, à contre-courant dudit mélange gazeux, ledit second conduit possédant une ouverture d'entrée (310) et une ouverture d'évacuation (320); (iii) une membrane (400) réalisée en un polymère hydrophobe et perméable uniquement pour ledit constituant et formant une paroi de séparation entre lesdits premier et second conduits, et (iv) un couple d'électrodes formées respectivement par une électrode de référence (330) et une électrode de mesure (340), disposées dans ledit conduit (300) aux extrémités de la membrane (400).

13. Dispositif selon l'une quelconque des revendications 7 à 12, caractérisé en ce que ledit second conduit (300) comprend des parties en acier inoxydable qui portent une électrode de référence (330) constituée par une première partie de surface oxydée et stabilisée, et une électrode de mesure (340) constituée par une seconde partie de surface oxydée et stabilisée, ladite électrode de mesure (340) étant plus longue de 20 à 50 % que l'électrode de référence (330), de 2 à 5 fois plus.
